# EUROPEAN PATENT APPLICATION

(11) **EP 0 672 380 A1**
(43) Date of publication of application: **20.09.1995**
(21) Application number: 95830091.5
(22) Date of filing: 10.03.1995
(51) Int. Cl.: A61B 5/00

(54) **Apparatus for measuring the temperature of the epidermis**

(30) Priority: 15.03.1994 IT BO940104
(71) Applicant: EUROPIANA S.r.l., I-40127 Bologna (IT)
(72) Inventor: Cappello, Angelo, I-40138 Bologna (IT); Piana, Lorenzo, I-40024 Castel San Pietro Terme(Bologna) (IT)
(74) Representative: Lanzoni, Luciano

(57) **Abstract**

The apparatus for measuring the temperature (T) of the epidermis (E) comprises temperature reading means (1) consisting of a plurality of heat-sensitive elements (11) uniformly distributed on a surface (S) that may be fitted closely to a portion of epidermis (PE), the distribution being such as to define a coordinate system; the elements (11) are connected to a processor (4) designed to process the signal corresponding to the measured temperature (T) and at least one ordered pair of values (X, Y) defining the position of one of the said elements (11) and to generate an output signal Vᵢ accordingly, in such a way as to generate a map (M) which visualizes the distribution pattern of the temperature (T) on the said portion of epidermis (PE).

## Description

The present invention relates to an apparatus for measuring the temperature of the epidermis to be used preferably in the field of medicine, especially for dermatological, cosmetic and related applications.

Many of the therapeutic and preventive treatments currently used require knowledge of epidermal temperature distribution and therefore means to measure this quantity prior to applications of various kinds.

In addition, to test many dermatological products skin reaction is monitored by measuring surface temperature following application of the products themselves.

One method of measuring the distribution of skin temperature is projection thermography or thermovision, consisting basically of a telecamera with an infrared-sensitive tube connected to a picture tube which makes it possible to visualize the thermal radiation emitted by the body, and especially by a portion of the epidermis.

This measurement method has the disadvantage of complexity and is not viable in certain sectors, for example, in beauty parlours, where personnel carrying out the examination do not usually have the technical skill required to operate an apparatus of this kind. Moreover, the costs of such an apparatus may be disproportionately high in relation to its purpose.

Various methods of measuring body temperature have been disclosed, for example, by patent publications US 4524778, FR 2516779 and BE 830008 where body temperature is determined by using liquid crystals, although the aims are different from those of the present invention. According to the inventions disclosed in the aforesaid patent publications, liquid crystals are placed in contact with parts of the body either directly or indirectly and changes in the colour of the crystals indicate the temperature of the corresponding part of the body.

One of the disadvantages of these thermographic methods is that the only way of recording the images provided by the liquid crystals is to photograph them.

The aim of the present invention is to eliminate the disadvantages by providing an easy to use, accurate and relatively low-cost measuring apparatus.

The invention, as characterized in the claims below, achieves this aim by providing an apparatus for determining temperature distribution comprising means for reading the temperature consisting of a plurality of heat-sensitive elements uniformly distributed on a surface that can be fitted closely to a portion of the epidermis, the distribution being such as to define a coordinate system; the heat-sensitive elements are connected to a processor designed to output a signal in accordance with the temperature detected by the heat-sensitive elements, the position of each element being defined by at least one ordered pair of values, so as to form a pattern representing the temperature distribution on the said portion of epidermis.

By associating appropriate recording means to the said processor, the present invention may be advantageously used to file or otherwise store the temperature patterns obtained. This makes it possible to create a "thermographic archive" containing the temperature distribution patterns of different persons examined during different stages of diagnosis or treatment.

The data accumulated in this way may be processed with a computer which means that the "map" generated by the temperature and coordinate signals may, for example, be broken down into the portions of major interest, enlarged, colour-coded and subjected to all the operations permitted by computer technology.

The technical characteristics and advantages of the invention are apparent from the detailed description which follows, with reference to the accompanying drawings, which illustrate a preferred embodiment of the invention by way of example and in which:
- Figure 1 is a schematic representation of an embodiment of the present invention, in which the different components are not to scale in order to better illustrate the invention as a whole;
- Figure 2 is a schematic representation of an embodiment of the present invention applied to a part of the body where the temperature is to be measured.

With reference to the drawings listed above, an apparatus for measuring the epidermal temperature T made in accordance with the present invention comprises first means 1 for reading the temperature of the epidermis E, appropriately applied to a portion of the epidermis. In the embodiment described, the reading means 1 consist of heat-sensitive elements 11 mounted on a flat, flexible element 10 which may be fitted closely to a portion of epidermis PE.

In particular, the heat-sensitive elements 11 may be of the semiconductor type, that is to say, they may consist of heat-sensitive diodes or transistors mounted on a band 10 of elastomeric material. If the elements are semiconductor devices, the diodes (or transistors) may, after being fitted to the band 10, be calibrated in a temperature bath so as to guarantee a uniform response to thermal stimulation on the entire surface of band 10.

The heat-sensitive elements 11 may also consist of other types of temperature sensors, for example, RTDs, thermistors or thermocouples; in practice, any sensor capable of converting a temperature input signal into an output signal corresponding to the variation of a parameter (resistance, capacitance, etc.) of a voltage or electric current.

The heat-sensitive elements 11 are connected to second means 2 for transmitting the electrical signal corresponding to the temperature T to third means 3 for displaying the data corresponding to the temperature T. The display means 3 consist preferably of at least one visual display unit.

The plurality of heat-sensitive elements 11 is uniformly distributed on a surface S (which, in the embodiment illustrated consists of the inner face of the band 10) which fits closely to a portion of the epidermis PE, the distribution being such as to define a coordinate system.

The surface S fits closely to a portion of the epidermis PE in that it may consist of the inside face of a strip or band (which may have fastening means, such as, for example, hooks or Velcro) as illustrated in the drawings or, in an embodiment that is not illustrated, it may form the lining of a portion of a rigid structure shaped in such a way as to complement the shape of the part of the body whose temperature is to be measured and applied so as to make the surface S correspond to the portion of epidermis PE concerned.

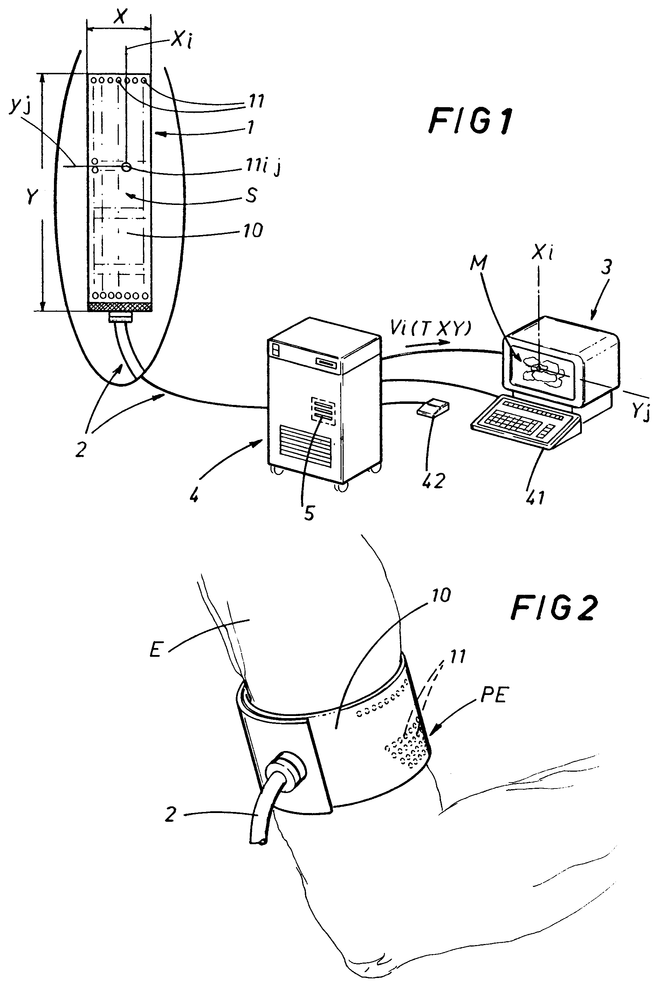

In the embodiment illustrated in Fig. 1, the heat-sensitive elements 11 are arranged on a rectangular base surface in such a way as to define a cartesian coordinate system.

Obviously, the heat-sensitive elements may be distributed differently, for example, in such a way as to form a polar coordinate system. For this invention, the important factor lies in being able to exactly locate each single heat-sensitive element 11 on the total surface S in such a manner as to combine the ordered pair of values Xᵢ, Yⱼ (coordinates) defining the location of the single element 11 with the temperature T measured by that element. The temperature measured, therefore, may be associated to a matrix defined by the pairs of values constituting the coordinates of the single heat-sensitive element 11.

Between the aforesaid transmitting means 2, which may be constituted by electrical cables, and the display means 3, there are located fourth means 4 designed to process the signals corresponding to the measured temperature T and the X, Y coordinates of each single heat-sensitive semiconductor element 11 and to generate an output signal Vᵢ accordingly. The transmitting means 2 may, either together with the processor 4, or independently of it, define a system for addressing the temperature readings taken. In practice, when readings are taken, the heat-sensitive elements 11 are selected by scanning the mounting surface in any of various ways, according to the distribution of the elements themselves; for example, if they are arranged in a cartesian system, first the lines and then the columns may be scanned, and the resulting data transmitted to the processor 4. The output data of the heat-sensitive elements 11 are a set of parameters or electrical quantities each of which is a function of a reference value and of the temperature reached by the single element. Obviously, to process this set of output values and reference input values, appropriate coding/decoding and multiplexing means are envisaged to send the signals to the processor 4.

In this way, it is possible to create a map M on the display unit 3 which visualizes the distribution of the temperature T on the portion of epidermis PE.

The processor 4 may consist of a personal computer 4 (which, as illustrated in Fig.1, may have a keyboard 41 and a mouse 42) which runs appropriate software and which may be equipped with, or connected to, fifth means 5 for recording data, consisting of a hard disk, a floppy disk or other data storage medium.

The signals Vᵢ supplied by the processor 4 are filed in the data storage medium 5 so that readings taken by the heat-sensitive elements 11 at different times are available for processing when required. As said above, therefore, this makes it possible to create a "thermographic archive" containing the temperature distribution maps of different persons examined during different stages of diagnosis or treatment.

The display means 3, need not be a visual display unit but may also be a printer, a plotter, or other peripheral device capable of representing the temperature readings in a suitable manner.

The invention described can be subject to modifications and variations without thereby departing from the scope of the inventive concept. Moreover, all the details of the invention may be substituted by technically equivalent elements.

## Claims

**1)** An apparatus for measuring the temperature (T) of the epidermis (E) comprising first means (1) for reading the temperature (T) of the epidermis, second means (2) for transmitting the electrical signal generated by the said reading means (1) as a function of the said temperature (T) to third means (3) for displaying the data corresponding to the temperature (T), characterized in that the said reading means (1) consist of a plurality of heat-sensitive elements (11), uniformly distributed on a surface (S) that may be fitted closely to a portion of epidermis (PE), the distribution being such as to define a coordinate system, and characterized also in that between the transmitting means (2) and the display means (3) there are located fourth means (4) designed to process the signal corresponding to the measured temperature (T) and at least one ordered pair of values (X, Y) defining the position of one of the said heat-sensitive elements (11) and to generate an output signal Vᵢ accordingly, in such a way as to generate a map (M) on the display means (3) which visualizes the distribution pattern of the temperature (T) on the said portion of epidermis (PE).

**2)** The apparatus for measuring the temperature (T) of the epidermis (E) according to claim 1, characterized in that the said heat-sensitive elements (11) are mounted on a flat, flexible element (1) which may be fitted closely to a portion of epidermis (PE).

**3)** The apparatus for measuring the temperature (T) of the epidermis (E) according to claim 1, characterized in that the said first reading means (1) consist of heat-sensitive semiconductor elements.

**4)** The apparatus for measuring the temperature (T) of the epidermis (E) according to claim 1, characterized in that the said heat-sensitive elements (11) consist of heat-sensitive diodes mounted on a flat, flexible element consisting of a band (10) of elastomeric material.

**5)** The apparatus for measuring the temperature (T) of the epidermis (E) according to claim 1, characterized in that the said heat-sensitive elements (11) consist of heat-sensitive transistors mounted on a flat, flexible element consisting of a band (10) of elastomeric material.

**6)** The apparatus for measuring the temperature (T) of the epidermis (E) according to claim 1, characterized in that the said fourth processing means (4) may be equipped with or connected to fifth data recording means (5) designed to store the signals (V) supplied by the processor (4) in such a way as to allow readings taken by the first reading means (1) at different times to be processed at a later stage.

**7)** The apparatus for measuring the temperature (T) of the epidermis (E) according to claim 1, characterized in that the distribution of the said heat-sensitive elements (11) is such as to define a cartesian coordinate system.

**8)** The apparatus for measuring the temperature (T) of the epidermis (E) according to claim 1, characterized in that the distribution of the said heat-sensitive elements (11) is such as to define a polar coordinate system.
